Europäisches Patentamt

European Patent Office

Office européen des brevets

(11) Numéro de publication: **0 318 106**
**A1**

(12) **DEMANDE DE BREVET EUROPEEN**

(21) Numéro de dépôt: 88202639.6

(22) Date de dépôt: 23.11.88

(51) Int. Cl.4: **A61B 17/22**

(30) Priorité: 24.11.87 FR 8716256

(43) Date de publication de la demande:
31.05.89 Bulletin 89/22

(84) Etats contractants désignés:
**DE FR GB NL**

(71) Demandeur: **PHILIPS SYSTEMES MEDICAUX**
**177, rue de Bezons**
**F-78420 Carrières sur Seine(FR)**

(84) **FR**

Demandeur: **N.V. Philips'**
**Gloeilampenfabrieken**
**Groenewoudseweg 1**
**NL-5621 BA Eindhoven(NL)**

(84) **DE GB NL**

(72) Inventeur: **Jatteau, Michel**
**Société Civile S.P.I.D.**
**209 rue de l'Université F-75007 Paris(FR)**

(74) Mandataire: **Landousy, Christian et al**
**Société Civile S.P.I.D. 209, Rue de**
**l'Université**
**F-75007 Paris(FR)**

(54) **Appareil de destruction de concrétions dans un milieu ou un organisme.**

(57) Appareil de destruction de concrétions dans un milieu ou un organisme, comprenant un dispositif (300) d'émission d'ondes à l'extérieur dudit milieu ou organisme et de focalisation de ces ondes à l'emplacement des concrétions repéré à l'aide d'un dispositif de localisation. Ce dispositif de localisation comprend un ensemble fluoroscopique (100) de détermination d'un axe $S_1 L_1$ de situation de la concrétion à détruire et un ensemble (200) de détection des photons diffusés transversalement audit axe de situation. L'ensemble fluoroscopique (100) comprend une source (101) de rayons X et un diaphragme (111), ainsi qu'un système (102) de traitement de signaux et de calcul de coordonnées. L'ensemble (200) de détection des photons diffusés comprend un détecteur de photons et un circuit électronique (204) de comptage et éventuellement de spectrométrie des scintillations détectées.

Application : lithotriteurs médicaux

FIG.1

# APPAREIL DE DESTRUCTION DE CONCRETIONS DANS UN MILIEU OU UN ORGANISME

La présente invention concerne un appareil de destruction de concrétions dans un milieu ou un organisme, comprenant un dispositif d'émission d'ondes à l'extérieur dudit milieu ou organisme et de focalisation de ces ondes à l'emplacement desdites concrétions, ledit emplacement étant repéré à l'aide d'un dispositif de localisation.

Un tel appareil, encore appelé lithotriteur, permet notamment la destruction de calculs, ou lithiases, à l'intérieur du corps humain. Les lithiases sont, on le rappelle, le résultat de la formation de divers types de concrétions dans différents organes ou dans leurs voies excrétrices, et les plus fréquentes sont celles des voies urinaires et des voies biliaires.

Dans les lithotriteurs dits extracorporels, des ondes de choc sont engendrées à l'extérieur du patient puis concentrées sur la lithiase au moyen d'un dispositif de focalisation. L'objectif poursuivi est d'obtenir, mais seulement à l'endroit de la cible pour éviter d'endommager les tissus voisins, des pressions suffisantes pour provoquer la fragmentation ou l'érosion de cette concrétion. Il est donc impératif de disposer, dans l'appareil, d'un système de localisation précise des concrétions. Il est d'ailleurs non seulement indispensable de connaître avec précision l'emplacement des concrétions à détruire, mais aussi très utile de pouvoir suivre leurs éventuels déplacements provoqués soit par les chocs subis au cours du traitement soit par les mouvements des organes, par exemple au cours de la respiration ou de contractions musculaires du patient.

Le brevet US-A- 4 617 931 décrit un appareil à impulsions ultrasonores pour la destruction de calculs (ou lithiases), dans lequel, tout en engendrant des ondes de choc au moyen d'un générateur d'impulsions ultrasonores à transducteur sphérique, on prévoit également un générateur auxiliaire d'ultrasons permettant d'effectuer facilement la localisation du calcul. Un appareil de ce type présente cependant une résolution spatiale limitée à quelques millimètres, en raison du principe mis en jeu (longueur d'onde des ultrasons dans la gamme des applications médicales). Il peut en outre être difficile de repérer les échos correspondant au calcul lui-même parmi ceux qui sont dûs aux structures biologiques voisines de celui-ci, et relativement long de devoir effectuer une analyse successive de coupes voisines pour découvrir le ou les plans contenant ce calcul.

Le but de l'invention est de proposer un appareil de destruction de concrétions qui, tout en restant d'un coût relativement modéré, bénéficie d'une qualité d'image et d'une résolution spatiale supérieures à celles de la réalisation citée.

A cet effet, l'appareil selon l'invention est caractérisé en ce que le dispositif de localisation comprend un ensemble fluoroscopique de détermination d'un axe $S_1 L_1$ de situation de la concrétion à détruire et un ensemble de détection des photons diffusés transversalement audit axe de situation pour la localisation précise de la concrétion sur cet axe, ledit ensemble fluoroscopique comprenant lui-même d'une part une source de rayons X et un diaphragme placé devant le tube à rayons X de ladite source permettant de passer d'un fonctionnement classique à pleine ouverture de ce diaphragme à une irradiation à ouverture réduite de la tranche de milieu ou organisme contenant la concrétion, ou réciproquement, et d'autre part un système de traitement de signaux et de calcul de coordonnées.

La structure du dispositif de localisation ainsi proposé remédie aux inconvénients mentionnés plus haut. En effet, la combinaison de l'action de l'ensemble fluoroscopique, pour la localisation de l'axe où se trouve la concrétion, et de l'action du détecteur de position transversal permet d'obtenir une résolution spatiale très satisfaisante. Cette résolution, de l'ordre du millimètre ou même inférieure, permet de repérer même les fragments résiduels obtenus pendant ou après la destruction de la concrétion.

Il existe, certes, des lithotriteurs munis d'un système de localisation à rayons X grâce auquel, à partir de deux images prises simultanément sous deux incidences différentes au moyen de deux ensembles fluoroscopiques, on peut connaître la position de lithiases, à l'intersection de deux droites correspondant à ces deux incidences, et focaliser en conséquence les ondes destructrices. Un exemple d'un tel appareil est décrit par exemple dans la demande de brevet WO 85/03631.

Ces lithotriteurs présentent cependant des inconvénients majeurs. En effet, si le premier ensemble fluoroscopique peut être considéré comme un équipement de base, permettant en outre aux urologues de pratiquer des examens et interventions par voie endoscopique, l'utilisation d'un deuxième ensemble similaire d'une part alourdit le coût de l'appareil et d'autre part conduit le patient à absorber un rayonnement important, du fait de l'irradiation par deux tubes radiogènes durant tout ou partie du traitement. L'utilisation d'un seul ensemble fluoroscopique sous deux incidences successives serait certes possible, mais elle ne permet pas de travailler en temps réel. Par ailleurs, la technique utilisée, à un ou deux ensembles fluoroscopiques, conduit ici à de faibles contrastes pour l'image du calcul, en raison d'une part de la superposition des images des tissus et organes environnants et d'autre part de la présence de nombreux photons diffusés. La structure selon l'invention évite ces inconvénients.

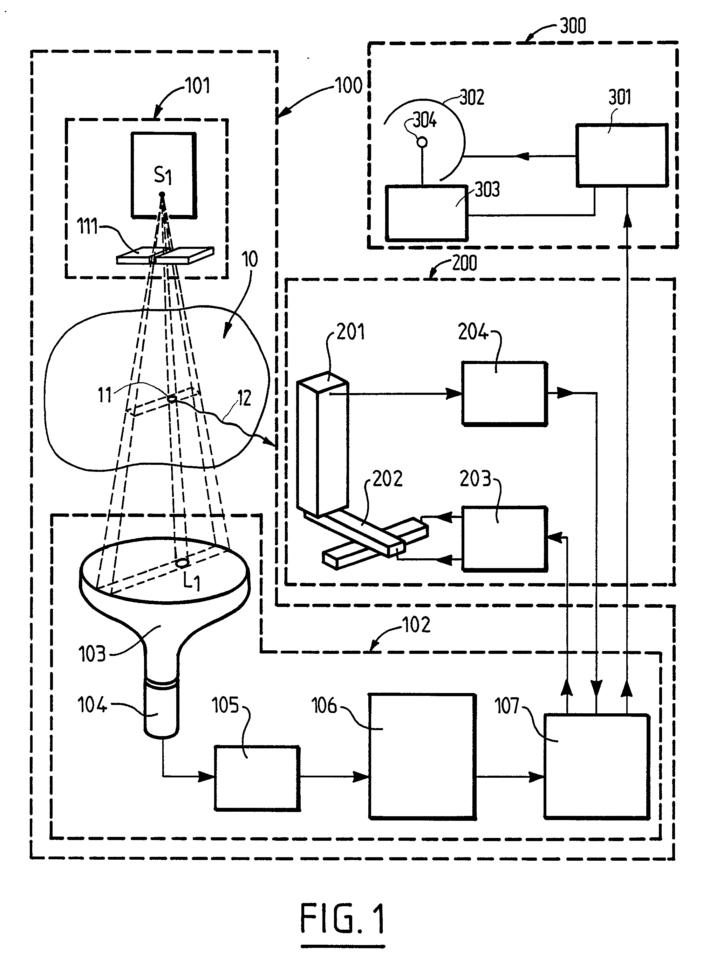

Les particularités et avantages de cette invention apparaîtront maintenant de façon plus précise dans la description qui suit et sur la figure 1, donnée à titre d'exemple non limitatif de réalisation du dispositif de localisation équipant l'appareil selon l'invention.

Le dispositif de localisation représenté sur la figure 1 comprend tout d'abord un ensemble fluoroscopique 100 et d'autre part un ensemble 200 de détection de photons. L'ensemble 100 comprend lui-même une source 101 de rayons X, composée essentiellement d'un générateur de haute tension et d'un tube à rayons X à collimateur, et un système 102 de traitement de signaux et de calcul de coordonnées.

Ce système 102, situé par rapport à la source 101 de l'autre côté du milieu 10 à examiner, comprend successivement un tube intensificateur d'image 103, une caméra de télévision 104, des circuits électroniques de traitement 105, un moniteur d'affichage d'images vidéo 106, et un calculateur 107. Au tube à rayons X est associé un diaphragme 111 pouvant être disposé de façon à n'irradier que la tranche du milieu 10 contenant la concrétion 11, après repérage préalable de celle-ci par formation d'une image classique obtenue à pleine ouverture du diaphragme.

L'ensemble de détection de photons 200 comprend un jeu 201 de sondes de détection à scintillateur, étroitement collimaté pour ne détecter que les photons X diffusés dans une mince tranche du milieu 10. Ce jeu de sondes 201 est automatiquement orienté, à l'aide d'un mécanisme d'asservissement linéaire ou circulaire 202, sur la tranche de milieu contenant la droite sur laquelle est située la concrétion 11 à détruire (cette droite $S_1L_1$ est représentée sur la figure 1). Un module de commande 203 placé en sortie du calculateur 107 fournit au mécanisme 202 les informations relatives à la position de la droite $S_1L_1$, déterminée par le calculateur 107 à partir de l'image vidéo et de la position de l'origine $S_1$ du faisceau de rayons X.

La concrétion 11, plus opaque aux rayons X que les tissus qui l'environnent, est le siège d'un rayonnement diffusé 12. La localisation précise de ce rayonnement 12 est effectuée par l'une des sondes du jeu 201, celle à laquelle correspond un maximum de photons détectés. Chacune de ces sondes est par exemple constituée d'un scintillateur et d'un photomultiplicateur associé à un circuit électronique 204 de comptage et éventuellement de spectrométrie des scintillations détectées (l'utilisation de la spectrométrie revenant ici à effectuer un filtrage en énergie), et la position de la concrétion 11 sur la droite $S_1L_1$ est donnée par la sonde qui fournit le taux de comptage le plus élevé. Le calculateur 107, qui reçoit d'une part la sortie du moniteur 106 et d'autre part celle du circuit électronique 204, délivre alors les coordonnées tridimensionnelles de la concrétion 11 à détruire, à partir desquelles est commandé un dispositif d'émission d'ondes destructrices 300. Le dispositif d'émission 300 comprend lui-même un circuit de commande 301 qui reçoit les coordonnées de la concrétion 11, et qui commande l'asservissement de la position d'un dispositif de focalisation 302 ainsi que le fonctionnement d'un générateur 303 associé à un transducteur 304 pour engendrer des ondes de choc. Le dispositif de focalisation 302 focalise les ondes de choc à l'emplacement de la concrétion 11.

Bien entendu, la présente invention n'est pas limitée au mode de réalisation préférentiel ci-dessus décrit et représenté, à partir duquel des variantes peuvent être proposées sans pour cela sortir du cadre de l'invention.

De telles variantes concernent essentiellement la réalisation de l'ensemble 200 de détection de photons. En effet, au lieu d'utiliser le jeu de sondes 201, on peut ne prévoir qu'une seule sonde de détection étroitement collimatée et associée à un dispositif de balayage mécanique le long de la droite $S_1L_1$ prédéterminée. On peut aussi utiliser, comme dispositif de détection, un seul cristal scintillateur en barreau, associé à un arrangement linéaire de tubes photomultiplicateurs. Dans ce cas, la localisation des scintillations implique un calcul barycentrique (le dispositif de détection est alors équivalent à une caméra de gammagraphie de type Anger à une seule dimension).

Par ailleurs, au lieu de sondes à scintillateur et tube photomultiplicateur, on peut utiliser des sondes de détection à semiconducteur, tel le tellure de cadmium CdTe. Le détecteur de photons diffusés peut encore être réalisé à partir d'un détecteur linéaire de scintillations à chambre à fils. Un tel détecteur convient bien pour la gamme d'énergie concernée par les photons diffusés et présente l'avantage d'une résolution spatiale élevée.

On notera enfin que le collimateur du détecteur peut être à trous parallèles ou à trous préformés pour obtenir un effet de focalisation dans un plan ou de collimation focalisée en un point (avec, alors, association à un système de balayage mécanique). En contrepartie d'une moindre rapidité de localisation, on accroît ainsi la sensibilité de détection.

## Revendications

1. Appareil de destruction de concrétions dans un milieu ou un organisme, comprenant un dispositif d'émission d'ondes à l'extérieur dudit milieu ou organisme et de focalisation de ces ondes à l'emplacement desdites concrétions, ledit emplacement étant repéré à l'aide d'un dispositif de localisation, caractérisé en ce que le dispositif de localisation comprend un ensemble fluoroscopique de détermination d'un axe $S_1 L_1$ de situation de la concrétion à détruire et un ensemble de détection des photons diffusés transversalement audit axe de situation pour la localisation précise de la concrétion sur cet axe, ledit ensemble fluoroscopique comprenant lui-même d'une part une source de rayons X et un diaphragme placé devant le tube à rayons X de ladite source permettant de passer d'un fonctionnement classique à pleine ouverture de ce diaphragme à une irradiation à ouverture réduite de la tranche de milieu ou organisme contenant la concrétion, ou réciproquement, et d'autre part un système de traitement de signaux et de calcul de coordonnées.

2. Appareil selon la revendication 1, caractérisé en ce que l'ensemble de détection de photons comprend un jeu de sondes de détection à scintillateur, équipé d'un collimateur limitant la zone de détection à une mince tranche dudit milieu ou organisme et associé à un dispositif de balayage mécanique commandé par le système de traitement et de calcul.

3. Appareil selon la revendication 2, caractérisé en ce que le collimateur est un collimateur focalisé.

4. Appareil selon la revendication 1, caractérisé en en ce que l'ensemble de détection de photons comprend une seule sonde de détection à scintillateur, cette sonde étant équipée d'un collimateur limitant la zone de détection à un seul axe de détection et étant associée à un dispositif de balayage mécanique commandé par le système de traitement et de calcul.

5. Appareil selon l'une des revendications 1 à 4, caractérisé en ce que l'ensemble de détection de photons comprend une ou plusieurs sondes de détection à semiconducteur.

6. Appareil selon l'une des revendications 1 à 3, caractérisé en ce que l'ensemble de détection de photons comprend un seul scintillateur en barreau, associé à un arrangement linéaire de tubes photomultiplicateurs et à un calculateur de localisation des scintillations de type barycentrique.

7. Appareil selon l'une des revendications 1 à 3, caractérisé en ce que l'ensemble de détection de photons comprend un détecteur de scintillations à chambre à fils.

FIG.1

PHF 87-595

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | US-A-4 179 100  (D. SASHIN)<br>* Colonne 4, lignes 43-53; colonne 4, ligne 61 - colonne 5, ligne 16; colonne 9, ligne 60 - colonne 10, ligne 9; colonne 14, lignes 7-32; colonne 15, lignes 5-11,41-56; colonne 15, ligne 62 - colonne 16, ligne 14; figures 1a,7,16a,16b,21 * | 1-6 | A 61 B   17/22 |
| Y | | 7 | |
| | --- | | |
| Y | FR-A-2 302 587  (G.E.C.)<br>* Figure 1; revendication 1 * | 7 | |
| | --- | | |
| A | DE-A-2 639 631  (F. AURICH)<br>* Figure 1; page 5, lignes 1-17 * | 1 | |
| | --- | | |
| A,D | EP-A-0 148 653  (J. DORY)<br>* Résumé; figure 1 * | | |
| | ----- | | |

**DOMAINES TECHNIQUES RECHERCHES (Int. Cl.4)**

A 61 B

Le présent rapport a été établi pour toutes les revendications

| Lieu de la recherche | Date d'achèvement de la recherche | Examinateur |
|---|---|---|
| LA HAYE | 28-02-1989 | NEILL M.C. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

 

& : membre de la même famille, document correspondant

EPO FORM 1503 03.82 (P0402)